# EUROPEAN PATENT APPLICATION

(11) **EP 4 275 682 A1**
(43) Date of publication of application: **15.11.2023**
(21) Application number: 22305704.3
(22) Date of filing: 12.05.2022
(51) Int. Cl.: A61K 31/28, A61K 31/555, A61P 1/00

(54) **CARBON MONOXIDE-RELEASING MOLECULE (CORM) OR COMPOSITION THEREOF FOR USE IN THE TREATMENT OR THE PREVENTION OF AN INTESTINAL DYSBIOSIS IN A SUBJECT**

(71) Applicant: INSTITUT NATIONAL DE LA SANTE ET DE LA RECHERCHE MEDICALE - INSERM, 75013 Paris (FR); Université Paris-Est Créteil Val de Marne, 94000 Créteil (FR)
(72) Inventor: MOTTERLINI, Roberto, 75010 PARIS (FR); FORESTI, Roberta, 75010 PARIS (FR)
(74) Representative: Cabinet Beau de Loménie

(57) **Abstract**

The invention concerns a carbon monoxide-releasing molecule (CORM) or composition thereof for use in the treatment or the prevention of an intestinal dysbiosis in a subject. More particularly, the invention concerns a carbon monoxide-releasing molecule (CORM) or composition thereof for use in the treatment or the prevention of an intestinal dysbiosis, preferably a caecum and/or colon dysbiosis in a subject, wherein the carbon monoxide-releasing molecule or composition thereof is administered orally.

## Description

### FIELD OF THE INVENTION

The invention relates to a carbon monoxide-releasing molecule (CORM) or composition thereof for use in the treatment or the prevention of an intestinal dysbiosis in a subject.

### BACKGROUND OF THE INVENTION

The mammal gastrointestinal microbiota consists in 10¹² to 10¹⁴ microorganisms such as bacteria, viruses, or even eukaryotes as fungi or yeasts which colonize the digestive tract soon after birth and live in the digestive tract of mammals [1]. The gastrointestinal microbiota is mainly located in the small intestine and the colon, distributed between the lumen of the digestive tract and the protective biofilm formed by the intestinal mucus which covers its inner wall [2]. Due to the gastric acidity, the stomach harbors a hundred million times less commensal bacteria than the colon. For this reason, most of the time it is referred to gut microbiota or intestinal microbiota.

The intestinal microbiota of human beings is progressively build up from birth, in contact with maternal and environmental bacteria during birth and continues to be populated through feeding and other contacts, followed by a gradual bacterial colonization which takes place in a specific order. It is established that bacterial content of human gut microbiota is composed mainly from Firmicutes, Bacteriodetes, Actinobacteria, Proteobacteria, Fusobacteria and Archaea with a predominance of Firmicutes and Bacteriodetes [3].

The use of high-throughput sequencing methods has made possible to characterize all the microbial genomes found in the intestine and to identify a thousand of different species among which bacteria represent the major part.

The intestinal microbiota is qualitatively and quantitatively unique to each human being with some common features. Among the 160 species of bacteria found on average in the microbiota of a healthy individual, only half are commonly found from one individual to another. However, there exists a common base of 15 to 20 bacterial species present in all human beings, in charge of the essential functions of the microbiota [2].

The first intestinal bacteria that colonize the gut are aerobic bacteria such as Enterococci, Staphylococci, etc. which need oxygen to proliferate. By consuming the oxygen present in the intestine, aerobic bacteria then promote the implantation of anaerobic bacteria such as Bacteroides, Clostridium, Bifidobacterium, Lactobacillus etc. which do not require oxygen to proliferate or in some cases, only proliferate in the absence of oxygen.

The composition of the intestinal microbiota will evolve qualitatively and quantitatively during the first years of life, depending on various factors such as dietary diversification, genetics, levels of hygiene, medical treatments received and the environment. This composition then remains quite stable, even if this stability seems to vary from one person to another.

With more than 1014 microorganisms covering the entire surface of the digestive tract, essentially in the intestine, the intestinal microbiota acts as a barrier against aggressive agents, by competing with pathogens for nutrients and binding sites, producing inhibitory substances or by preventing their penetration into the intestinal mucosa. Additionally, the microbial genome encodes 3 to 4 million genes, which is approximately 150 times more than the human genome and allows the microbiota's microorganisms to perform several metabolic activities that are not encoded by the human genome [1].

A dynamic of mutual benefits exists between the microorganisms from the intestinal microbiota and the host organism, which results in the maintenance of normal immunological, metabolic and motor functions, and even a correct nutrient digestion and absorption [4].

Considering the importance of a healthy intestinal microbiota for the maintenance of various biological functions, an intestinal dysbiosis, meaning a quantitative and/or a qualitative and/or a functional alteration of the intestinal microbiota, may cause or favour various physiological troubles or diseases.

During lifetime, several factors may have an impact more or less important, on the composition of the intestinal microbiota and cause or at least favour an intestinal dysbiosis. Among these factors we can list ageing, various diseases, medical treatments, lifestyle or diet changes, or even pesticides or additives present in food.

An intestinal dysbiosis refers to an imbalance in the intestinal microbial community leading to a qualitative and/or a qualitative and/or a functional alteration of intestinal microbiota. Considering the importance of a healthy intestinal microbiota for the maintenance of various physiological functions, it is crucial to maintain a healthy intestinal microbiota during lifetime or to be able to recover a healthy intestinal microbiota after a temporary dysbiosis.

Prebiotic and probiotic compositions are known and are often proposed to maintain or restore the intestinal microbiota. Probiotics are viable microorganisms which confer health benefits to the host when administered in sufficient amount and prebiotics are typically non-digestible fermentable ingredients that induce specific changes in the composition and/or activity of the resident microflora by selectively augmenting the proliferation of bacteria responsible for exerting beneficial effects upon host well-being and health[5] [6].

The inventors interestingly propose a new approach for restoring or preserving the intestinal microbiota of a subject to a healthy state. Therefore, the present invention refers to a carbon monoxide-releasing molecule (CORM) or a composition thereof for use in the treatment or the prevention of an intestinal dysbiosis in a subject, preferably a human being.

Indeed, the inventors have shown that oral administration to a subject of a carbon monoxide-releasing molecule (CORM) or composition thereof, allows increasing and/or decreasing the abundance of one or more bacterial species, especially anaerobic bacterial species, present in the intestinal microbiota of a subject, relative to the abundance of the bacterial species present in the intestinal microbiota of a subject prior to administering the CORM or composition thereof.

These results are surprising as they demonstrate the ability of a carbon monoxide-releasing molecule (CORM) or composition thereof to go through the gastrointestinal tract and to specifically reach and accumulate in the intestines, wherein it exerts beneficial effects on the intestinal microbiota.

Various studies have been made to understand the role of carbon monoxide in many physiological and pathological processes and to identify potential uses of carbon monoxide-releasing molecules for the treatment or the prevention of some physiological dysfunctions or even diseases. However, it is the first time that a carbon monoxide-releasing molecule (CORM) or a composition thereof is proposed for its use in the treatment or prevention of an intestinal dysbiosis in a subject, preferably a human.

### SUMMARY OF THE INVENTION

As mentioned above, the inventors interestingly propose a new approach for restoring or preserving the intestinal microbiota composition of a subject in a healthy state. Interestingly, the inventors have shown that the oral administration of a carbon monoxide-releasing molecule (CORM), allows reshaping the intestinal microbiota towards a healthy phenotype, especially by increasing and/or decreasing the abundance of some specific bacterial species present in the intestinal microbiota.

Thus, a first object of the invention concerns a carbon monoxide-releasing molecule (CORM) or composition thereof for use in the treatment or the prevention of an intestinal dysbiosis in a subject, wherein the carbon monoxide-releasing molecule or composition thereof is administered orally or via rectal route.

A second object of the invention concerns a carbon monoxide-releasing molecule (CORM) or a composition thereof for use in a method for the treatment or the prevention of an intestinal dysbiosis in a subject, wherein the carbon monoxide-releasing molecule or a composition thereof is administered orally.

A third object of the invention concerns a carbon monoxide-releasing molecule (CORM) or a composition thereof for use in a method for restoring and/or maintaining the intestinal microbiota of a subject in a healthy state, wherein the carbon monoxide-releasing molecule or a composition thereof is administered orally.

### Legends to the figures

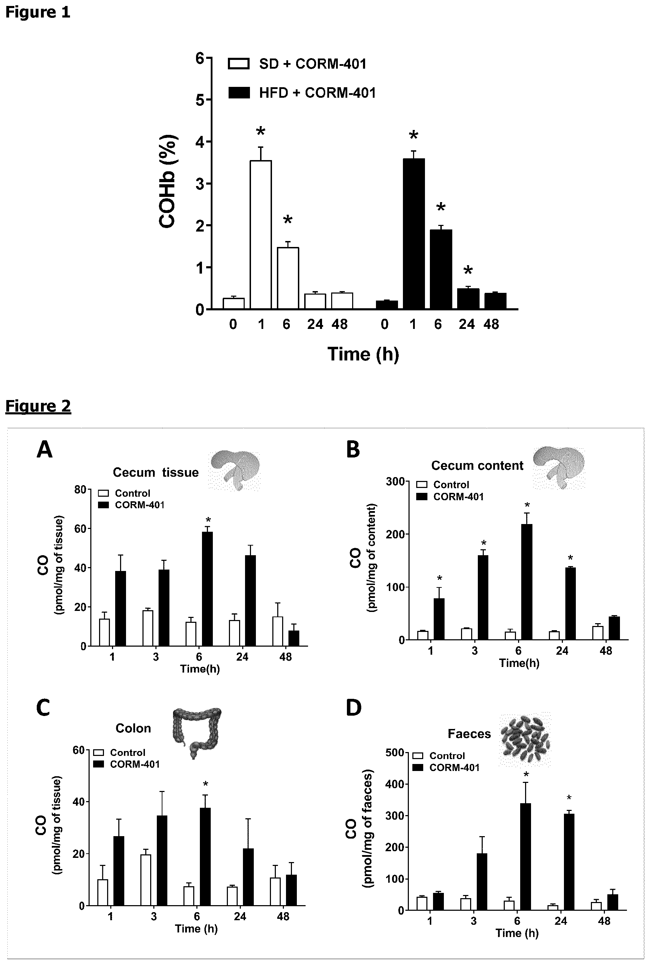

**Figure 1** shows the levels of blood carbon monoxide hemoglobin (COHb) after oral administration of CORM-401 in mice fed a high fat diet over a period of 48 hours. Statistical analysis was performed using one-way Anova followed by the Fisher multi-comparison test. Values are expressed as mean±S.E.M. ^{∗}p<0.01 vs. time 0;
**Figure 2** shows the time-dependent CO accumulation in tissues of the gastrointestinal tract such as caecum (A) and colon (C) as well as in the caecum content (B) and faeces (D) in the in HFD mice orally administered with CORM-401 or with PBS as control. Statistical analysis was performed by a student t-test. Values are expressed as mean±S.E.M. ^{∗}p<0.01 vs. control.

### DETAILED DESCRIPTION OF THE INVENTION

### Definitions

Carbon monoxide (CO) is known as an air pollutant but also as an endogenous signalling molecule which may play a critical role in many physiological and pathological processes. Carbon monoxide is endogenously produced in many living organisms as a result of the breakdown of heme by the enzyme heme oxygenase, which exists in constitutive (HO-2 and HO-3) and inducible (HO-1) isoforms. Most of the CO is bound to hemoglobin with the formation of COHb while the remaining CO is distributed in tissues, exerting several physiological functions such as vasodilatory, anti-inflammatory, anti-proliferation effects. Considering the difficulty to store CO and to control its dosage in case of a therapeutic use, a variety of carbon monoxide-releasing molecules has been developed [7].

The terms "Carbon monoxide-releasing molecules (CORMs)" refer to chemical compounds designed to release controlled amounts of carbon monoxide (CO). CORMs are typically categorized in metal CORMs (also known as "metal carbonyl CORMs) and non-metallic CORMs. The CORM can release carbon monoxide spontaneously, or by contacting, for example, a suitable solvent or medium, such as an aqueous physiological fluid (e.g., blood or lymph), or an aqueous physiological cellular material, such as a tissue, organ, or cell. A CORM also can release carbon monoxide by irradiation. For example, the CORM can be irradiated either prior to administration to produce a solution of dissolved CO, or in situ after administration

Metal CORMs are compounds that contain a transition metal such as nickel, cobalt, ruthenium, manganese, molybdenum, rhodium, boron or iron surrounded by carbonyl (CO) groups as coordinated ligands [8] and which are able to release CO in biologic systems. The metal CORMs can also include additional ligands that may modulate a particular property of the CORM, such as, for example, the rate of releasing carbon monoxide, solubility, hydrophobicity, stability, or electrochemical potential. The additional ligands can be, for example, halides, sulfoxides, natural and synthetic amino acids, aromatics, carboxylates, ethers, alcohols, or nitriles. Metal CORMs comprise carbonmonoxy myoglobin (CO-Mb), carbonmonoxy hemoglobin (CO-Hb) and polyethylene glycol-conjugated hemoglobin (CO-MP4).

Non-metallic CORMs are metal-free carbon monoxide-releasing molecules with different chemical structures, among them we can refer to boranocarbonates such as CORM-A1, Diels-Alder reaction-based CO donors, 3-hydroxyflavone (3-HF), quinolone, cyclic diketone, xanthene-9-carboxylic acid, *meso*-carboxyl BODIPY derivatives etc [7]. Non-metallic CORMs comprise organic CO-releasing molecules, such as oxalic acid (CAS number: 144-62-7), cyclopentenone derivatives, cyclopentadienone-alkyne pairs and S-aryl thioformates.

In the context of the invention, the CORM may be a salt such as a pharmaceutically acceptable salt.

The term "pharmaceutically acceptable salt" refers to salts of acids or bases, known for their use in the preparation of active principles for their use in therapy. Examples of pharmaceutically acceptable acids suitable as source of anions are those disclosed in the Handbook of Pharmaceutical Salts: Properties, Selection and Use (P. H. Stahl and C. G. Wermuth, Weinheim/Zürich:Wiley-VCH/VHCA, 200). Salts that are approved by a regulatory agency of the Federal or a state government or listed in the U.S. or European Pharmacopeia or other generally recognized pharmacopeia for use in animals, and humans. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, 6 hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts.

As used herein, the terms "microbiota" (also known as or "microflora") refer to the community of microorganisms, such as bacteria, archaea, protists, fungi, and viruses, that typically inhabits a bodily organ or part.

The intestinal microbiota corresponds to the community living microorganisms which inhabit the intestinal tract, mainly in the small intestine and the colon, including the caecum which connects the colon to the small intestine.

The "caecum" corresponds to the first part of the colon. It is made up of a pouch into which the orifice of the ileo-caecal valve opens. It is at this valve that the small intestine, especially the ileum empties into the colon. The term "colon" refers to the longest part of the large intestine and it is localized between the caecum and the rectum. In the human caecum and the colon, 10¹² CFU/mL or greater of bacteria may be found, mainly anaerobic bacterial species.

The term "Anaerobic bacterial species" refer to bacterial species which are oxygen-sensitive bacterial species. These terms include (i) strict anaerobic bacterial species, which are extremely sensitive to oxygen and must not be in contact with oxygen to grow, (ii) aerotolerant anaerobic bacterial species, which can be exposed to a low concentration of oxygen without stopping their growth, and (ii) facultative anaerobic bacterial species which are able to grow both in presence or in absence of oxygen.

The terms "Aerobic bacterial species" refer to bacterial species that survive and grow only in presence of oxygen in their environment.

The term "dysbiosis" corresponds to a physiological state in which a subject displays a microbiota profile that differs or deviates significantly from a corresponding microbiota profile that is typical of a normal (healthy) subject, such as a change in the microbiota commensal species diversity and/or quantity as compared to a normal (healthy) subject. It is possible to diagnose a dysbiosis and to measure the extent of a dysbiosis by comparing how different a microbiota profile is from the microbiota profile of a normal (healthy) subject. A typical microbiota profile of a normal (healthy) subject may be obtained from a single subject or even a single sample from a single subject, but preferably will be obtained from a plurality of subjects and the techniques used will allow for intra-individual variation. Comparison between the profile from a test sample and that of a normal (healthy) reference in order to assess whether the test sample is dysbiotic or not and, optionally, the extent of any dysbiosis may be achieved by any convenient means and the choice of approach used may be dictated by the form of the data making up the profiles under comparison. Depending on the nature of the data, the comparison may be a qualitative, semi-quantitative or quantitative process. Various methods exist to determine the presence of a dysbiosis, especially trough different indexes based on comparison to a set of individuals or samples used as references.

The term "intestinal dysbiosis" refers to an imbalance in the intestinal microbial community leading to a quantitative and/or qualitative and/or a functional alteration of intestinal microbiota in comparison with the corresponding microbiota of a normal (healthy) subject. Conveniently, the intestinal dysbiosis status of a sample from a test subject may be assessed using the Dysbiosis Index test described in WO 2016/156251. This test can analyse intestinal microbiota profiles from test samples in comparison to those from healthy subjects and apply a relative score proportional to the extent of dysbiosis in the sample. Thus a sample may be classified as being dysbiotic or not and the extent of any dysbiosis may also be determined. Changes in the dysbiotic/normobiotic status of a subject can therefore be easily monitored before, during and/or after treatment.

The term "caecum and/or colon dysbiosis" refers to an imbalance in the caecum and/or colon microbial community leading to a quantitative and/or qualitative and/or a functional alteration of caecum and/or colon microbiota in comparison with the corresponding microbiota of a control subject. The control subject may correspond to a normal, healthy, subject or a subject that does not have the same condition as the one who suffers from caecum and/or colon dysbiosis.

The term "treating" or "treatment" means reversing, alleviating, inhibiting or slowing the progress of the disorder or condition to which such term applies, or one or more symptoms of such disorder or condition, in a subject.

The term "preventing" or "prevention" means decreasing the risk that a subject develops the disorder or condition to which such term applies, or one or more symptoms of such disorder or condition.

The terms "microbiota healthy state", "microbiota normal state" or "microbiota state of health" correspond to the typical profile of the microbiota of a normal, healthy, subject, which may also be referred as "normobiosis" or a "normobiotic state". The "microbiota healthy state" corresponds to a microbiota wherein the microbiota quantity and/or diversity correspond to the one of a healthy subject, meaning who does not suffer from a dysbiosis.

The term "healthy subject" or "normal subject" refers to a subject who does not suffer from an intestinal dysbiosis.

The term "subject", "patient" or "individual", as used herein can be used interchangeably and refers to a human, non-human mammal (such as a rodent (mouse, rat), a feline, a canine, or a primate), birds and fish affected or likely to be affected with an intestinal dysbiosis. Preferably, the subject is a human, man or woman.

The term "high fat diet" refers to a diet consisting of at least 35% of total calories consumed from fats, both unsaturated and saturated.

The term "abundance" refers to the amount of the microorganism to which such term applies. The abundance of a microorganism in the intestinal microbiota may be measured by extracting DNA and RNA from stool samples followed by a sequencing carried out by metagenomic/metatranscriptomic technique as previously described in literature [9].

The term "pharmaceutically acceptable" refers to approved by a regulatory agency of the Federal or a state government or listed in the U.S. or European Pharmacopeia or other generally recognized pharmacopeia for use in animals, and humans.

The term "pharmaceutical composition" refers to a composition comprising pharmaceutically acceptable carrier and a "nutraceutical composition" means a composition comprising a physiologically acceptable carrier. For example, a carrier can be a diluent, adjuvant, excipient, or vehicle with which the therapeutic is administered. Such carriers can be sterile liquids, such as water and oils, including those of petroleum, animal, vegetable or synthetic origin, such as peanut oil, soybean oil, mineral oil, sesame oil and the like. Suitable pharmaceutical/physiological excipients include starch, glucose, lactose, sucrose, sodium stearate, glycerol monostearate, talc, sodium chloride, dried skim milk, glycerol, propylene glycol, water, ethanol and the like. In the context of the invention, the pharmaceutical composition or the nutraceutical composition is adapted for oral administration. For instance, the tablets or capsules, can be prepared by conventional means with pharmaceutically acceptable excipients such as binding agents (e.g. pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. For instance, liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. According to the invention, the pharmaceutical or nutraceutical composition may be a capsule, tablet, powder, pill, sugar-coated pill, granule, sachet, gel, paste, syrup, emulsion, suspension, suppository, solution and the like.

The term "nutraceutical" refers to ordinary food that has components or ingredients added to give it a specific medical or physiological benefit, other than a purely nutritional effect. It comprises the dietary supplements and the functional foods. It may be in a liquid form, a solid form, a powder form, such as pills, tablets, capsules, granules, gels, pastes, syrups, emulsions, suspensions, solutions, drinks, soups, nutritional bars, confectionery, milk-based products or fermented milk-based products, yoghurts, milk-based powders, enteral nutritional products, infant and/or baby compositions, fermented or non-fermented cereal-based products, ice creams, chocolate, coffee, "culinary" products such as mayonnaise, tomato puree or salad dressings.

### Invention

A first object of the invention relates to a carbon monoxide-releasing molecule (CORM) or composition thereof for use in the treatment or the prevention of an intestinal dysbiosis in a subject, wherein the carbon monoxide-releasing molecule or composition thereof is administered orally or via rectal route.

A second object of the invention relates to a carbon monoxide-releasing molecule (CORM) or a composition thereof for use in a method for the treatment or the prevention of an intestinal dysbiosis in a subject, wherein the carbon monoxide-releasing molecule or a composition thereof is administered orally.

A third object of the invention concerns a carbon monoxide-releasing molecule (CORM) or a composition thereof for use in a method for restoring and/or maintaining the intestinal microbiota of a subject in a healthy state, wherein the carbon monoxide-releasing molecule or a composition thereof is administered orally.

The terms "restoring" or "reshaping" encompasses any positive effect on the intestinal dysbiosis of the subject. As such not only is complete normalisation of the intestinal microbiota profile covered (i.e. a return to normobiosis or a "normalised" or normal intestinal microbiota profile) but partial improvement in the dysbiosis of the subject or even an improvement of the intestinal microbiota profile. Improvement may be partial in the sense that perturbations in the abundance of, or the metabolic phenotypes of, a subset of the microorganisms or groups of microorganisms in the profile are improved (preferably normalised) and/or that the extent of perturbation in the abundance of, or the metabolic phenotypes of, particular microorganisms or groups of microorganisms is partially reduced. Preferably the intestinal microbiota of a subject which has intestinal dysbiosis is (fully) normalised.

The term "maintain" encompasses the substantially continuous persistence of a normal intestinal microbiota profile of a subject, or part thereof, during a period of administration of effective amounts of the CORM or composition thereof. Therefore, significant perturbation in the abundance of, or the metabolic phenotypes of, one or more microorganisms or groups of microorganisms does not occur.

In some embodiments, the CORM is selected from:
- metal carbonyls, such as CORM-401 (CAS number: 1001015-18-4), CORM-2 (CAS number: 22594-69-0), CORM-3 (CAS number: 475473-26-8), CORM-371, EBOR-CORM-1, ALF-186, ALF-850 and ALF-021, carbonmonoxy myoglobin (CO-Mb), carbonmonoxy hemoglobin (CO-Hb) and polyethylene glycol-conjugated hemoglobin (CO-MP4);
- metal-free CO-releasing compounds, such as CORM-A1 (CAS number: 17363-08-5); and/or
- organic CO-releasing molecules, such as oxalic acid (CAS number: 144-62-7), cyclopentenone derivatives, cyclopentadienone-alkyne pairs and S-aryl thioformate.

The transition metal carbonyls EBOR-CORM1, CORM-371, ALF-186, ALF-850 and ALF-021[7] have the following chemical formula and/or structure:

**Table 1**

| Compound name | Chemical formula and/or structure |
|---|---|
| EBOR-CORM 1 | [NEt₄][MnBr₂(CO)₄] |
| CORM-371 | 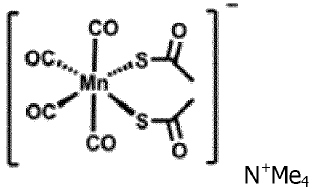 |
| ALF-186 | 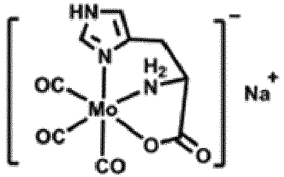 |
| ALF-850 | 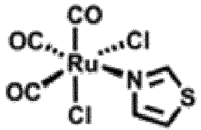 |
| ALF-021 | 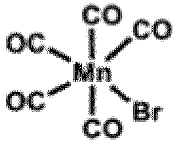 |

For instance, the cyclopentadienone-alkyne pairs may be selected from: BW-CO-104, BW-CO-105, BW-CO-106, BW-CO-108, BW-CO-109, BW-CO-110, BW-CO-111, BW-CO-112 and/or BW-CO-113 as disclosed in Zhixiang Pan et al, "Organic CO-prodrugs: Structure CO-release rate relationship Studies", Chem. Eur. J. 10.1002/chem.201700936. The S-aryl thioformate may be selected from the list disclosed in De Simone et al, "ThioCORMates: Tunable and Cost-Effective Carbon Monoxide-Releasing Molecules", Chemistry, 2022 May 9. doi: 10.1002/chem.202201326 [14] and more specifically from the S-thioformate compounds listed in Table 2 here below

These compounds have the following chemical formula and/or structure:

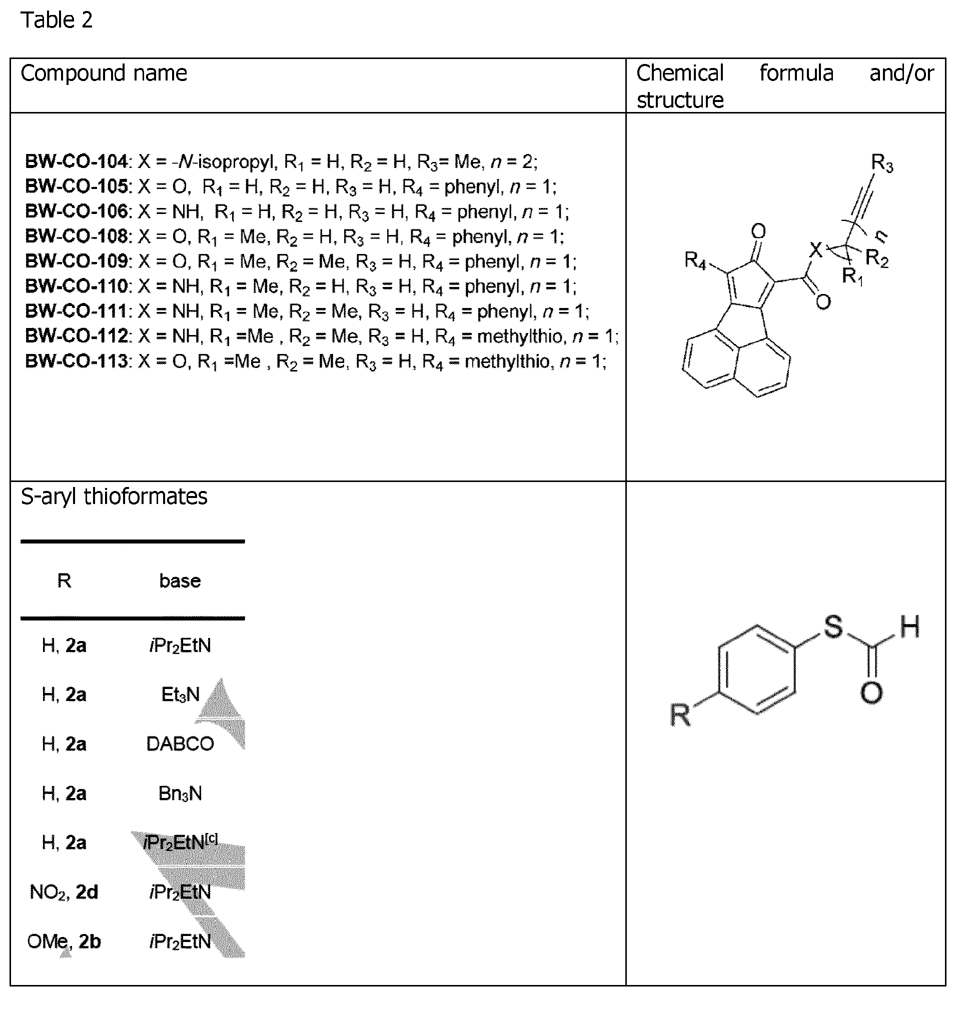

In some embodiments, the carbon monoxide-releasing molecule is chosen among CORM-401, CORM-A1 or a mixture thereof.

In some embodiments, the carbon monoxide-releasing molecule is CORM-401 (CAS number: 1001015-18-4).

In some embodiments, the CORM refers to a chemically modified CORM, such as a CORM conjugated to copolymers, or a CORM modified for being sensitive to a specific enzyme, light radiation or other stimuli.

In some embodiments, the carbon monoxide-releasing molecule is chosen among carbonmonoxy myoglobin (CO-Mb), carbonmonoxy hemoglobin (CO-Hb) and polyethylene glycol-conjugated hemoglobin (CO-MP4).

In some embodiments, the CORM is conjugated to copolymers, such as polyethylene glycol. An example of CORM conjugated to polyethylene glycol is the polyethylene glycol-conjugated hemoglobin called "CO-MP4".

In some embodiments, the subject is a human.

In some embodiments, the intestinal dysbiosis is a caecum and/or colon dysbiosis, preferably a colon dysbiosis.

Thus, according to a specific embodiment of the invention, the CORM or composition thereof is administered to the subject before, during and/or after a high fat diet or a drug therapy inducing dysbiosis, such as antibiotic treatment. According to a preferred aspect, the CORM or composition thereof is administered to the subject at least during a high fat diet or a drug therapy inducing dysbiosis, such as antibiotic treatment, preferably before, during and after a high fat diet or a drug therapy inducing dysbiosis, such as antibiotic treatment.

For instance, the antibiotic treatment may be chosen among clindamycin, vancomycin, dalbavancin, oritavancin, telavancin, neomycin, sulfasalazine, ampicillin, phenoxymethylpenicillin, flucloxacillin, tobramycin, metronidazole, tinidazole, gentamicin, bacitracin, streptomycin, amoxicillin, cephalexin, lymecycline, tetracycline, deoxycycline, ciprofloxacin, levofloxacin, norfloxacin, erythromycin, azithromycin, clarithromycin, co-trimoxazole, nitrofurantoin, trimethoprim, sulfamethoxazole, amikacin, erythromycin, fidaxomicin, gemifloxacin, moxifloxacin, delafloxacin, cefuroxime, ceftriaxone, ceftazidime, ceftaroline, cefotetan, cefotaxime, cefiderocol, cefepime, cefdinir, omadacycline, minocycline, eravacycline, demeclocycline, sarecycline, imipenem, cilastatin, meropenem, ertapenem. In some embodiments, the abundance of one or more bacterial species is increased and/or decreased relative to the abundance of the bacterial species prior to administering the CORM or composition thereof.

In some embodiments, the CORM or composition thereof for use according to the invention promotes the recovery or favour the return of the intestinal microbiota of a subject to a normal, healthy state. The CORM or composition thereof according to the invention may decrease the abundance of potential harmful bacterial species and/or increase of the abundance of potential beneficial bacterial species present in the intestinal microbiota, in comparison with the abundance of these bacterial species prior to the administration of the CORM or composition thereof to a subject suffering from an intestinal dysbiosis.

In some embodiments, the CORM or composition thereof decreases the risk that the abundance of potential harmful bacteria species increases and/or that the abundance of potential beneficial bacteria species present in the intestinal microbiota decreases.

In some embodiments, the CORM or composition thereof for use according to the invention restores the intestinal microbiota of the subject in a healthy state.

In some embodiments, the CORM or composition thereof for use according to the invention maintains the intestinal microbiota of the subject in a healthy state.

In a more particular embodiment, the abundance of one or more bacterial species is normalized relative to the abundance of the one or more of the bacterial species prior to administering the CORM or composition thereof or maintained to a normal abundance.

In some embodiments, the abundance of one or more bacterial species is increased or decreased by 2 times, by 3 times or by 4 times relative to the abundance of the bacterial species prior to administering the CORM or composition thereof.

Methods for measuring the relative abundance of intestinal microbiota in a sample include but are not limited to nucleic acid analysis (e.g. nucleic acid sequencing approaches, oligonucleotide hybridisation probe based approaches, primer based nucleic acid amplification approaches), antibody or other specific affinity ligand based approaches, proteomic and metabolomic approaches. The analysis of ribosomal RNA genes and the transcripts thereof, e.g. the 5S, 16S and 23S genes (preferably the 16S gene) in prokaryotic microorganisms or the 18S gene in eukaryotic microorganisms, may be convenient.

In some embodiments, the bacterial species is one or more of the bacterial species present in the intestinal microbiota. For example, the bacterial species are selected among one or several of the following bacterial species: *Akkermansia muciniphila, Alistipes fingoldii, Alistipes shahii, Olsenella umbonata, Olsenella uli, Faecalibaculum rodentium, Lactobacillus murinus, Lactobacillus fermentum, Libanicoccus massiliensis, Lactobacillus salivarius, Lactobacillus agilis, Lactobacillus ruminis, Lactobacillus animalis, Parabacteroides goldsteinii, Aerococcus viridans, Rombutsia ilealis, Lactococcus lactis, Enterococcus faecium, Streptococcus thermophiles, Enterococcus faecalis, Lactococcus piscium, Carnobacterium divergens, Listeria monocytogenes.*

In some embodiments, the CORM or composition thereof for use according to the invention normalizes or maintains normal of abundance of one or more, e.g. at least 2, 3, 4, 5 or all of these bacterial species, preferably in a human subject.

The bacterial species *Akkermansia muciniphila, Alistipes fingoldii, Alistipes shahii, Olsenella umbonata, Olsenella uli, Faecalibaculum rodentium, Lactobacillus murinus, Lactobacillus fermentum, Libanicoccus massiliensis, Lactobacillus salivarius, Lactobacillus agilis, Lactobacillus ruminis, Lactobacillus animalis, Parabacteroides goldsteinii, Aerococcus viridans* and especially the bacterial specie *Akkermansia muciniphila,* are among the most abundant bacterial species present in the intestinal microbiota of a healthy, normal subject. The abundance of one or more of these bacterial species in the intestinal microbiota of a subject suffering from an intestinal dysbiosis, especially in the context of a high fat diet or a drug therapy inducing dysbiosis, may be decreased.

In some embodiments, the abundance of one or more anaerobic bacterial species selected from *Akkermansia muciniphila, Alistipes fingoldii, Alistipes shahii, Olsenella umbonata, Olsenella uli, Faecalibaculum rodentium, Lactobacillus murinus, Lactobacillus fermentum, Libanicoccus massiliensis, Lactobacillus salivarius, Lactobacillus agilis, Lactobacillus ruminis, Lactobacillus animalis, Parabacteroides goldsteinii, Aerococcus viridans* is increased relative to the abundance of these anaerobic bacterial species prior to administering the CORM or composition thereof.

In a specific embodiment, the abundance of one or more anaerobic bacterial species selected from *Akkermansia muciniphila, Alistipes fingoldii, Alistipes shahii, Olsenella umbonata, Olsenella uli* is increased relative to the abundance of these anaerobic bacterial species prior to administering the CORM or composition thereof.

In a specific embodiment, the abundance of the anaerobic bacterial specie *Akkermansia muciniphila* is increased relative to its abundance prior to administering the CORM or composition thereof.

The abundance of the bacterial species *Rombutsia ilealis, Lactococcus lactis, Enterococcus faecium, Streptococcus thermophiles, Enterococcus faecalis, Lactococcus piscium, Carnobacterium divergens, Listeria monocytogenes* and especially the bacterial specie *Romboutsia ilealis* in the intestinal microbiota of a subject suffering from an intestinal dysbiosis, especially in the context of a high fat diet or of a drug therapy inducing dysbiosis, is increased.

In some embodiments, the abundance of one or more anaerobic bacterial species selected from *Rombutsia ilealis, Lactococcus lactis, Enterococcus faecium, Streptococcus thermophiles, Enterococcus faecalis, Lactococcus piscium, Carnobacterium divergens and Listeria monocytogenes* is decreased relative to the abundance of these anaerobic bacterial species prior to administering the CORM or composition thereof. In particular, the abundance of *Romboutsia ilealis* in the intestinal microbiota of a subject suffering from an intestinal dysbiosis, especially in the context of a high fat diet or of a drug therapy inducing dysbiosis, is increased relative to the abundance of this anaerobic bacterial specie prior to administering the CORM or composition thereof.

In a preferred embodiment, the abundance of the anaerobic bacterial specie *Akkermansia muciniphila* is increased and the abundance of the anaerobic bacterial specie *Romboutsia ilealis* is decreased relative to the abundance of these anaerobic bacterial species prior to administering the CORM or composition thereof.

In some embodiments, the intestinal microbiota of a subject suffering of an intestinal dysbiosis is restored or reshaped towards a healthy phenotype.

In a particular embodiment, the carbon monoxide-releasing molecule is administered at a dose from 1 mg/kg to 25 mg/kg, preferably from 2.5 mg/kg to 15 mg/kg.

The dose of carbon monoxide-releasing molecule (CORM) administered to a subject may vary depending on various factors such as the subject's age, weight and/or gender, the diet that the subject has followed, follows and/or will follow, the proportion of fat in the subject's diet, the drug therapy followed for instance the type of antibiotics used and/or the extent of the intestinal dysbiosis. The person skilled in the art will know how to determine the appropriate dose to be administered to a subject depending on these various factors, based on his knowledge in the field.

In some embodiments, the carbon monoxide-releasing molecule or composition thereof is administered 1 to 7 times a week, preferably 3 to 6 times a week.

In some embodiments, the carbon monoxide-releasing molecule (CORM) or composition thereof is administered to the subject during one week, during two weeks, during three weeks or more, such as during 1 month, during 2 months, during 3 months or even more if necessary, for example before, during and/or after a high fat diet or a drug therapy inducing dysbiosis, such as an antibiotic treatment.

In some embodiments, the carbon monoxide-releasing molecule (CORM) or composition thereof is administered daily during a high fat diet or a drug therapy inducing dysbiosis, such as an antibiotic treatment.

In some embodiments, the carbon monoxide-releasing molecule (CORM) or composition thereof is administered daily during at least three weeks, preferably during 1 month before a high fat or diet or a drug therapy inducing dysbiosis, such as an antibiotic treatment.

In some embodiments, the carbon monoxide-releasing molecule (CORM) or composition thereof is administered daily during at least three weeks, preferably during 1 month after a high fat diet or a drug therapy inducing dysbiosis, such as an antibiotic treatment.

In some embodiments, the carbon monoxide-releasing molecule (CORM) or composition thereof is administered daily during a high fat diet and during at least three weeks, preferably 1 month before and after a high fat diet or a drug therapy inducing dysbiosis, such as an antibiotic treatment.

In some embodiments, the CORM composition is a pharmaceutical composition or a nutraceutical composition.

In some embodiments, the CORM composition is made in a form suitable for oral or rectal use. The CORM composition can be in various forms including, for example, capsules, tablets, powders, pills, sugar-coated pills, granules, sachets, gels, pastes, syrups, emulsions, suspensions, solutions. Preferably, the CORM composition is in a form selected from the group consisting in capsules, tablets, powders, pills, sugar-coated pills, granules, suppositories. More preferably, the CORM composition is in a form of capsules or pills.

In some embodiments, the CORM or composition thereof is nanoencapsulated or contained in micelles.

In some embodiments, the nutraceutical composition is a food supplement, such as a dietary supplements or functional foods. The functional food may be a drink, a milk-based product, or a fermented milk-based product such as yoghurts.

The person skilled in the art will know how to determine the appropriate concentration of CORM to be comprised in the CORM composition depending on the dose of CORM to be administrated to the subject and on the form of the composition.

In some embodiments, the CORM composition comprises from 0,1% to 10% of CORM by weight relative to the total weight of the composition (w/w), such as from 1% to 5% w/w, for example 1% w/w or 3% w/w.

In some embodiments, a prebiotic and/or a probiotic are administered previously to, concomitantly with and/or subsequently to the CORM or composition thereof. The probiotic may be selected among one or more of the following bacterial species: *Akkermansia muciniphila, Eubacterium limosum, Parasutterella secunda, Lactobacillus sporogenes, Lactobacillus casei, Lactobacillus rhamnosus, Lactobacillus acidophilus, Bifidobacterium bifidum, Lactobacillus salivarius, Bifidobacterium infantis, Bifidobacterium longum, Bifidobacterium lactis, Lactobacillus johnsonii, Lactobacillus paracasei,* preferably *Akkermansia muciniphila, Eubacterium limosum, Parasutterella secunda.* The prebiotic may be selected among one or more of the following ingredients: dietary fibers from fruits, vegetables whole grains; fermented foods such as kombucha, kefir, skyr yogurt, fermented cottage cheese, vegetable brine drinks, and/or fermented vegetables, such as kimchi. Preferably, the prebiotic are chosen among skyr yogurt, vegetable brine drinks, fermented vegetables.

The invention will be further illustrated by the following figures and examples. However, these examples and figures should not be interpreted in any way as limiting the scope of the invention.

### EXAMPLES

### Example 1: Levels of blood carbon monoxide hemoglobin (COHb) after oral administration of CORM-401

### a) Material and method:

The levels of COHb (carbon monoxide bound to hemoglobin) were measured at different time points after oral administration of CORM-401 in two groups of eight-week-old male C57BL6 mice that were fed either a standard diet (SD) or a high fat diet (HFD) for 14 weeks (n=9-10 per group). CORM-401 was freshly prepared in phosphate buffer solution (PBS) and administered orally at a dose of 30 mg/kg=90 µmoles/kg (n=6) and COHb in blood was measured after 1, 6, 24 and 48 hours.

### b) Results

As shown in Figure 1, COHb levels in blood significantly increased in a similar fashion 1 hour after administration of CORM-401 in both SD-fed and HFD-fed mice and gradually decreased to baseline levels over a period of 48 hours. These data demonstrate that CO is released by CORM-401 and delivered into the blood circulation in a time-dependent fashion. Therefore, repetitive boosts of CO can be achieved every time this compound is orally administered to mice over the 14 week treatment.

These results confirm the rationale for giving CORM-401 three times a week based on the kinetic of CO delivery in mice by this compound.

### Example 2: Levels of CO accumulation in different intestinal tissues after oral CORM-401 administration

### a) Material and methods

Eight-week-old male C57BL6 mice weighing approximately 25 g were treated with an oral administration of either CORM-401 (30 mg/kg) or PBS (control group) and then sacrificed after 1, 3, 6, 24 and 48 hours (n=6 per time point). Organs were collected to determine the levels of CO accumulation in different intestinal tissues following the two treatments. The body compartments where the intestinal microbiota is most likely to be present such as the caecum, caecum content, colon and faeces were analyzed. CO accumulation was quantified using the CO sensitive scavenger hemoCD1 as previously reported by the inventors such as in Mao et al, 2021 [10]; Minegishi et al, 2017 [11]. Briefly, fifty milligrams of tissue were homogenized in 0.5 ml PBS in the presence of hemoCD1 (2-10 µM) and sodium dithionite (1 mg). Tissue homogenates were first sonicated, then samples were centrifuged, and the supernatants filtered before measurements by UV-VIS absorption spectroscopy. The concentration of CO was calculated from the absorbances at 422 and 434 nm using a reported equation (Kitagishi et al [12], 2010; Braud et al 2018 [13]).

### b) Results

It is observed that CO significantly accumulated in the caecum tissue (Figure 2A), caecum content (Figure 2B), colon (Figure 2C) and faeces (Figure 2D) in a time-dependent manner after CORM-401 treatment. The maximum CO content was reached 6 hours after administration of CORM-401, in all compartments, and returned to basal levels after 48 h. Thus, oral administration of CORM-401 to mice enables CO to be delivered and efficiently reach the tissues that directly interact with the intestinal microbiota.

### Example 3: Analysis of the impact of CORM-401 oral administration on the abundance of anaerobic bacterial species of intestinal microbiota

### a) Material and methods

Eight-week-old male C57BL6 mice weighing approximately 25 g were fed either a standard diet (SD), high fat diet (HFD) or HFD+CORM-401 for 14 weeks (n=9-10 per group). CORM-401 was prepared freshly in phosphate buffer solution (PBS) and administered orally to mice three times a week each time at a dose of (30 mg/kg=90 µmoles/kg) during the entire HFD regime. Faeces from the three groups of mice were then collected after the 14 week HFD regime for microbiota analysis before mice were sacrificed.

The stool samples DNA and RNA (n=3 per group) were extracted and the sequencing was carried out by a shotgun metagenomic/metatranscriptomic technique as previously described [9]. Briefly, a pre-extraction step combining mechanical, enzymatic and chemical procedures was performed prior to extraction by means of a QIAsymphony PowerFecal Pro DNA Kit (QIAGEN). The extracts were used for DNA/RNA library preparation and determination of pair-end sequences using a NextSeq500 Instrument (Illumina). Fastq files were analysed by an in-house software MetaMIC (v2.2.0). The data were filtered based on quality (phred score) and the sequences were mapped against several custom databases, counted and collected in a separate Fastq file. Sequence counts were made for each bacterial species and compared to the environmental control. The number of sequences for each species was used to determine their relative abundance in each group of mice.

### b) Results

As illustrated on Table 3, here below, the proportion of the 50 most frequent intestinal bacterial species present in SD-fed mice significantly changes after the 14-week HDF regime in the absence or presence of CORM-401. For example, the prototypic beneficial bacteria *Akkermansia muciniphila* is the second most abundant bacteria in SD-fed mice but does not appear among the most dominant bacteria in HFD-fed mice.

Interestingly, oral administration of CORM-401 reshapes the microbiota profile and restores the homeostasis of specific bacteria, which relative abundance is represented in Table 3. This microbiota profiling is confirmed when the relative abundance (number of DNA sequences) of each bacterial specie is measured in the three groups.

As shown in Table 3, HFD-fed mice had a significantly higher incidence of *Lactococcus lactis, Enterococcus faecium, Streptococcus thermophiles* and the potentially harmful bacteria species *Romboutsia ilealis* compared to SD-fed mice, while *Akkermansia muciniphila* was markedly decreased. Notably, HFD mice treated with CORM-401 displayed an intestinal bacteria profile that is characterized by *Akkermansia muciniphila* being among the most dominant species alongside a marked increase in the abundance of *Alistipes fingoldii, Alistipes shahii, Olsenella umbonata, Olsenella uli* and a decrease in the abundance of *Romboutsia ilealis.*

These results clearly indicate that oral administration of CORM-401 reshapes the intestinal microbiota HFD mice towards a healthy phenotype by increasing and/or decreasing the abundance of some specific anaerobic bacterial species present in the intestinal microbiota.

**Table 3: Impact of the oral administration of CORM-401 on the abundance and diversity of bacterial species found in the intestinal microbiota of HFD mice. Data are expressed in number of sequences (average of 3 independent experiments per group).**

| **Bacterial species** | **SD** | **HFD** | **HFD+CORM** |
|---|---|---|---|
| *Lactococcus lactis* | 16 | 1301700 | 642300 |
| *Muribaculum intestinale* | 329700 | 6200 | 21500 |
| *Lactobacillus johnsonii* | 40300 | 204100 | 162300 |
| *Lactobacillus reuteri* | 24500 | 258900 | 339700 |
| *Akkermansia muciniphila* | 61400 | 1100 | 120300 |
| *Faecalibaculum rodentium* | 12904 | 37456 | 120296 |
| *Lactobacillus gasseri* | 15025 | 75465 | 61826 |
| *Romboutsia ilealis* | 22 | 67303 | 21923 |
| *Bacteroides caecimuris* | 61060 | 10787 | 14812 |
| *Ruminiclostridium sp. KB18* | 13029 | 22981 | 18969 |
| *Burkholderiales bacterium YL45* | 24120 | 1472 | 159 |
| *Lachnoclostridium sp. YL32* | 12255 | 19807 | 14254 |
| *Lactobacillus fermentum* | 474 | 3521 | 10256 |
| *Enterococcus faecium* | 527 | 14793 | 7750 |
| *Streptococcus thermophilus* | 10 | 19798 | 7718 |
| *Bacteroides fragilis* | 13584 | 1917 | 3651 |
| *Bacteroides dorei* | 11885 | 1130 | 3093 |
| *Alistipes shahii* | 7737 | 2216 | 9490 |
| *Flavonifractor plautii* | 2747 | 7988 | 7720 |
| *Alistipes finegoldii* | 6827 | 1921 | 6727 |
| *Olsenella sp. Marseille-P2300* | 85 | 92 | 4915 |
| *Enterococcus faecalis* | 596 | 8793 | 4655 |
| *Lactococcus piscium* | 4 | 8040 | 4133 |
| *Lactococcus garvieae* | 5 | 6902 | 3953 |
| *Bacteroides xylanisolvens* | 6961 | 1064 | 1151 |
| *Adlercreutzia equolifaciens* | 1001 | 4481 | 4445 |
| *Lactobacillus murinus* | 219 | 751 | 4440 |
| *Libanicoccus massiliensis* | 75 | 61 | 4193 |
| *Faecalibacterium prausnitzii* | 2029 | 4579 | 3622 |
| *Clostridium bolteae* | 2926 | 4042 | 3559 |
| *Lactobacillus salivarius* | 218 | 619 | 3546 |
| *Lactobacillus helveticus* | 211 | 2342 | 2955 |
| *Listeria monocytogenes* | 65 | 4641 | 2783 |
| *Intestinimonas butyriciproducens* | 1046 | 2404 | 2426 |
| *Lachnoclostridium phocaeense* | 2512 | 3221 | 2366 |
| *Lactobacillus agilis* | 136 | 352 | 2223 |
| *Lactobacillus ruminis* | 192 | 397 | 2160 |
| *Erysipelotrichaceae bacterium I46* | 1010 | 2103 | 1788 |
| *Mordavella sp. Marseille-P3756* | 1638 | 1714 | 1240 |
| *Carnobacterium divergens* | 0 | 3387 | 1726 |
| *Aerococcus viridans* | 127 | 327 | 1650 |
| *Olsenella umbonata* | 45 | 41 | 1620 |
| *Olsenella uli* | 25 | 36 | 1483 |
| *Lactobacillus animalis* | 65 | 235 | 1355 |
| *uncultured Firmicutes bacterium* | 21 | 3236 | 1355 |
| *Roseburia intestinalis* | 1064 | 1862 | 1190 |
| *Clostridioides difficile* | 855 | 1480 | 1101 |
| *Oscillibacter valericigenes* | 452 | 1016 | 1053 |
| *Faecalitalea cylindroides* | 112 | 587 | 1039 |
| *Lactobacillus plantarum* | 16 | 1639 | 1011 |
| *Blautia sp. YL58* | 880 | 1343 | 934 |
| *Lactobacillus gallinarum* | 245 | 1129 | 928 |
| *Lactobacillus acidipiscis* | 40 | 122 | 630 |
| *Barnesiella viscericola* | 2436 | 128 | 350 |
| *Bacteroides cellulosilyticus* | 2126 | 127 | 246 |
| *Parabacteroides goldsteinii* | 17 | 4 | 43 |

### BIBLIOGRAPHIC REFERENCES

[1] Passos et al (2017), Intestinal microbiota in digestive diseases », Arq Gastroenterol, vol 54(3)
[2] https://www.inserm.fr/dossier/microbiote-intestinal-flore-intestinale/
[3] Suvoro et al, (2013), "Gut Microbiota, Probiotics, and Human Health", Biosci Microbiota Food Health., 32(3): 81-91
[4] Dave M. et al, (2012), "The human gut microbiome: current knowledge, challenges, and future directions", Transl Res., 160:246-57.
[5] Vandenplas et al, (2015), "Probiotics: an update", J Pediatr., 91(1):6-21.
[6] Devine et al, (2009), "Prospects for the development of probiotics and prebiotics for oral applications", J Oral Microbiol., 1: 10
[7] Cheng et al, (2021) « Recent Advances on Carbon Monoxide Releasing Molecules for Antibacterial Applications », ChemMedChem, 16, 3628-3634
[8] Herrmann WA., (1990), "100 Years of metal carbonyls: a serendipitous chemical discovery of major scientific and industrial impact", J Organomet Chem., 383:1-3
[9] Rodriguez C, et al, (2021), "Viral genomic, metagenomic and human transcriptomic characterization and prediction of the clinical forms of COVID-19", PLoS Pathog., 17(3): e1009416.
[10] Mao,Q., Kawaguchi,A.T., Mizobata,S., Motterlini,R., Foresti,R., & Kitagishi,H., (2021), "Sensitive quantification of carbon monoxide (CO) in vivo reveals a protective role of circulating hemoglobin in CO int"oxication. Commun. Biol. 4, 425-doi: 10.1038/s42003-021-01880-1.
[11] Minegishi,S., Yumura,A., Miyoshi,H., Negi,S., Taketani,S., Motterlini,R., Foresti,R., Kano,K., & Kitagishi,H, (2017), "Detection and removal of endogenous carbon monoxide by selective and cell permeable hemoprotein-model complexes". J. Am. Chem. Soc. 139, 5984-5991.
[12] Kitagishi,H., Negi,S., Kiriyama,A., Honbo,A., Sugiura,Y., Kawaguchi,A.T., & Kano,K., (2010). "A Diatomic Molecule Receptor That Removes CO in a Living Organism. Angew. Chem. Int. Ed Engl.", 49, 1312-1315.
[13] Braud,L., Pini,M., Muchova,L., Manin,S., Kitagishi,H., Sawaki,D., Czibik,G., Ternacle,J., Derumeaux,G., Foresti,R., & Motterlini,R. (2018), "Carbon monoxide-induced metabolic switch in adipocytes improves insulin resistance in obese mice.", JCI Insight 3, e123485
[14] De Simone et al, "ThioCORMates: Tunable and Cost-Effective Carbon Monoxide-Releasing Molecules", Chemistry, 2022 May 9. doi: 10.1002/chem.202201326.

## Claims

1. Carbon monoxide-releasing molecule (CORM) or composition thereof for use in the treatment or the prevention of an intestinal dysbiosis in a subject, wherein the CORM or composition thereof is administered orally or via rectal route.

2. CORM or composition thereof for use according to claim 1, wherein the CORM is selected from:
- metal carbonyls, such as CORM-401 (CAS number: 1001015-18-4), CORM-2 (CAS number: 22594-69-0), CORM-3 (CAS number: 475473-26-8), CORM-371, EBOR-CORM-1, ALF-186, ALF850, ALF021,
- carbonmonoxy myoglobin (CO-Mb), carbonmonoxy hemoglobin (CO-Hb) and polyethylene glycol-conjugated hemoglobin (CO-MP4);
- metal-free CO-releasing compounds, such as CORM-A1 (CAS number: 17363-08-5); and/or
- organic CO-releasing molecules, such as oxalic acid (CAS number: 144-62-7), cyclopentenone derivatives, cyclopentadienone-alkyne pairs and S-aryl thioformate.

3. CORM or composition thereof for use according to any of claims 1 or 2, wherein the carbon monoxide-releasing molecule is CORM-401 (CAS number: 1001015-18-4).

4. CORM or composition thereof for use according to 1 to 3, wherein the subject is a human.

5. CORM or composition thereof for use according to any of the preceding claims, wherein the intestinal dysbiosis is a caecum and/or colon dysbiosis.

6. CORM or composition thereof for use according to any of the preceding claims, wherein the CORM or composition thereof is administered before, during and/or after a dietary regimen that induces dysbiosis, such as high fat diet, or a drug therapy inducing dysbiosis, such as antibiotic treatment.

7. CORM or composition thereof for use according to any of the preceding claims, wherein the abundance of one or more bacterial species is increased and/or decreased relative to the abundance of the bacterial species prior to administering the CORM or composition thereof.

8. CORM or composition thereof for use according to any of the preceding claims, wherein the abundance of one or more bacterial species is normalized relative to the abundance of the one or more bacterial species prior to administering the CORM or composition thereof or maintained to a normal abundance.

9. CORM or composition thereof for use according to any of the preceding claims, wherein the abundance of one or more anaerobic bacterial species selected from *Akkermansia muciniphila, Alistipes fingoldii, Alistipes shahii, Olsenella umbonata, Olsenella uli, Faecalibaculum rodentium, Lactobacillus murinus, Lactobacillus fermentum, Libanicoccus massiliensis, Lactobacillus salivarius, Lactobacillus agilis, Lactobacillus ruminis, Lactobacillus animalis, Parabacteroides goldsteinii, Aerococcus viridans* is increased relative to the abundance of these anaerobic bacterial species prior to administering the CORM or composition thereof.

10. CORM or composition thereof for use according to any of the preceding claims, wherein the abundance of the anaerobic bacterial specie *Akkermansia muciniphila* is increased relative to its abundance prior to administering the CORM or composition thereof.

11. CORM or composition thereof for use according to any of the preceding claims, wherein the abundance of one or more anaerobic bacterial species selected from *Rombutsia ilealis, Lactococcus lactis, Enterococcus faecium, Streptococcus thermophiles, Enterococcus faecalis, Lactococcus piscium, Carnobacterium divergens andListeria monocytogenes* is decreased relative to the abundance of these anaerobic bacterial species prior to administering the CORM or composition thereof.

12. CORM or composition thereof for use according to any of the preceding claims, wherein the intestinal microbiota of a subject suffering of an intestinal dysbiosis is restored or reshaped towards a healthy phenotype.

13. CORM or composition thereof for use according to any of the preceding claims wherein the carbon monoxide-releasing molecule is administered at a dose from 1 mg/kg to 25 mg/kg, preferably from 2.5 mg/kg to 15 mg/kg.

14. CORM or composition thereof for use according to any of the preceding claims, wherein the carbon monoxide-releasing molecule is administered 1 to 7 times a week, preferably 3 to 6 times a week.

15. CORM or composition thereof for use according to any of the preceding claims, wherein the CORM composition is made in a form suitable for oral or rectal use.

16. CORM or composition thereof for use according to any of the preceding claims, wherein the CORM composition is a pharmaceutical composition or a nutraceutical composition.

17. CORM or composition thereof for use according to any of the preceding claims, wherein the CORM composition is selected from the group comprising of capsules, tablets, powders, pills, sugar-coated pills, granules, sachets, gels, pastes, syrups, emulsions, suspensions, suppositories, solutions.

18. CORM or composition thereof for use according to any of the preceding claims, wherein a prebiotic and/or a probiotic are administered previously to, concomitantly with and/or subsequently to the CORM or composition thereof.
